# EUROPEAN PATENT SPECIFICATION

(11) **EP 1 279 689 B1**
(45) Date of publication and mention of the grant of the patent: **01.10.2008**
(21) Application number: 02014069.5
(22) Date of filing: 01.07.2002
(51) Int. Cl.: C08G 61/12, C09K 19/34, C09K 19/38, H01B 1/12, C07D 333/12

(54) **Mono-, Oligo and Poly-3-(1,1-difluoroalkyl)thiophenes and their use as charge transport materials**
Mono-, Oligo and Poly-3-(1,1-difluoroalkyl)thiophene und ihre Verwendung als Ladungstransportmaterial
Mono-, Oligo et Poly-3-(1,1-difluoroalkyl)thiophènes et leur utilisation comme matériaux de transport de charges

(30) Priority: 25.07.2001 EP 01117651
(43) Date of publication of application: 29.01.2003
(73) Proprietor: Merck Patent GmbH, 64293 Darmstadt (DE)
(72) Inventor: Heeney, Martin, Southampton SO14 6TQ (GB); Farrand, Louise, Blandford Forum, Dorset DT11 9ED (GB); Giles, Mark, Southampton SO15 2LE (GB); Thompson, Marcus, Hampshire SP6 1RR (GB); Tierney, Steven, Southampton SO15 7QW (GB); Shkunov, Maxim, Southampton SO16 6SX (GB); Sparrowe, David, Bournemouth, Dorset BH6 5EJ (GB); McCulloch, Iain, Hampshire S020 6PE (GB)

(56) References cited:
- WO-A-02/09201
- RITTER S K ET AL: "SYNTHESIS, CHARACTERIZATION, AND OXIDATIVE POLYMERIZATION OF 3-(FLUOROMETHYL)THIOPHENES" CHEMISTRY OF MATERIALS, AMERICAN CHEMICAL SOCIETY, WASHINGTON, US, vol. 5, no. 6, 1993, pages 752-754, XP001149323 ISSN: 0897-4756
- BUCHNER W. ET AL.: "Synthesis of 3-(polyfluoroalkyl) thiophenes" JOURNAL OF FLUORINE CHEMISTRY., vol. 59, no. 3, 1992, pages 301-309, XP002236905 ELSEVIER SEQUOIA. LAUSANNE., CH ISSN: 0022-1139
- ROBITAILLE L ET AL: "SYNTHESIS, CHARACTERIZATION, AND LANGMUIR-BLODGETT FILMS OF FLUORINATED POLYTHIOPHENES" MACROMOLECULES, AMERICAN CHEMICAL SOCIETY. EASTON, US, vol. 27, no. 7, 28 March 1994 (1994-03-28), pages 1847-1851, XP000433198 ISSN: 0024-9297
- FACCHETTI A. ET AL.: "Tuning the semiconducting properties of sexithiophene by alpha, omega-substitution. Alpha, omega-diperfluorohexylsexithiophene: the first n-type sexithiophene for thin-film transistors" ANGEWANDTE CHEMIE. INTERNATIONAL EDITION., vol. 39, no. 24, 2000, pages 4547-4551, XP002236906 VERLAG CHEMIE. WEINHEIM., DE ISSN: 0570-0833

## Description

### Field of Invention

The invention relates to new mono-, oligo- and poly-3-(1,1-difluoroalkyl)thiophenes, polymerisable liquid crystal materials and anisotropic polymer film, including their oxidatively or reductively doped forms. The invention further relates to methods of their preparation, their use as semiconductors or charge transport materials in optical, electrooptical or electronic devices including field effect transistors, electroluminescent, photovoltaic and sensor devices. The invention further relates to field effect transistors and semiconducting components comprising the new mono-, oligo- and poly-3-(1,1-difluoro-alkyl)thiophenes. Furthermore the invention relates to a security marking or device and to a charge injection layer, planarising layer, antistatic film or conducting substrate or pattern.

### Background and Prior Art

Organic materials have recently shown promise as the active layer in organic based thin film transistors and organic field effect transistors (OFETs) [see reference 1]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semiconducting material and the current on/off ratio, so the ideal semiconductor should have a low conductivity in the off state, combined with a high charge carrier mobility (> 1 x 10⁻³ cm²V⁻¹ s⁻¹). In addition, it is important that the semiconducting material is relatively stable to oxidation i.e. it has a high ionisation potential, as oxidation leads to reduced device performance.

A known compound which has been shown to be an effective p-type semiconductor for OFETs is pentacene [see reference 2]. When deposited as a thin film by vacuum deposition, it was shown to have carrier mobilities in excess of 1 cm² V⁻¹ s⁻¹ with very high current on/off ratios greater than 10⁶. However, vacuum deposition is an expensive processing technique that is unsuitable for the fabrication of large-area films.

Regioregular poly(3-hexylthiophene) has been reported with charge carrier mobility between 1 x 10⁻⁵ and 4.5 x 10⁻² cm²V⁻¹ s⁻¹, but with a rather low current on/off ratio (10-10³) [see reference 3]. In general, poly(3-alkylthiophenes) show good solubility and are able to be solution processed to fabricate large area films. However, poly(3-alkylthiophenes) have relatively low ionisation potentials and are susceptible to doping in air [see reference 4].

Fluorinated poly(alkylthiophenes) were studied by L. Robitaille and M. Leclerc [see reference 5]. However, poly(3'-perfluorohexyl-terthiophene was found to be insoluble and poly[3-(tridecafluorononyl)thiophene] to be soluble in octafluorotoluene, a solvent unsuitable for large scale solution processing. Compared to its alkyl analogues, however, it exhibits inferior electronic properties, which was attributed to lower regioregularity. In addition, the bulky fluoroalkyl group dilutes the macroscopic charge transport mobility arising from the conjugated pi-electron component of the molecule. This large group also potentially disrupts the closely packed lammelar morphology, again lowering mobility.

Perfluoro-α-sexithiophene and α,ω-diperfluorohexylsexithiophene are described as potential n-type semiconductors [see references 6, 7]. In fact, the α,ω-substituted sexithiophene exhibits carrier mobilities of up to 2 x 10⁻² cm² V⁻¹ s⁻¹ for electron transport (*n*-type semi-conduction). However, both fluorinated sexithiophenes have low solubilities in common solvents, and therefore may not be solution processed.

A perfluoralkylated heteroaromatic polymer represented by the general formula 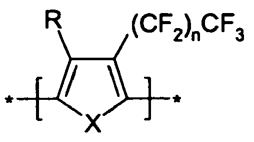 wherein R is H or alkyl, X is S, NH or O and n is 1 to 9, having a polymerization degree of 5 to 5000 is proposed in the EP 0 414 906 A1 [reference 8]. The polymer may be used as water- and oil-repellent and as an electrically conductive material. But again, the perfluoralkylated thiophenes show in general a low to very low solubility, especially in those organic solvents, which are commonly used in large scale production techniques.

S.K. Ritter et al., Chem. Mater. 1993, 5(6), p. 752-754; W. Buchner et al., Journal of Fluorine Chemistry 1992, 59(3), p. 301-309 and L. Robitaille et al., Macromolecules 1994, 27(7), p- 1847-1851 disclose polythiophenes with fluoroalkyl substituents, but do not disclose compounds as claimed in the present invention.

It is the aim of the present invention to provide new materials for use as semiconductors or charge transport materials, which are easy to synthesize, have high charge mobility, good processibility especially a good solubility, and a good oxidative stability.

Further aims of the present inventions relate to advantageous uses of the mono-, oligo- and polymers, including their oxidatively or reductively doped forms, according to the invention.

Other aims of the invention are immediately evident to those skilled in the art from the following description.

The inventors have found that these aims can be achieved by providing new oligomers and polymers based on regioregular 3-(1,1-difluoro-alkyl)thiophenes, especially with a head-to-tail orientation. These oligo- and polymers exhibit a high degree of planarity in the backbone and strong interchain π-π stacking interactions making them effective charge transport materials with high charge carrier mobilities. The electron withdrawing 1,1-difluoro group adjacent to the thiophene ring lowers the HOMO relative to the unfluorinated poly(3-alkyl)thiophenes. The oligo- and polymers according to the invention possess a sufficient high ionization potential and are sufficiently stable to oxidation. On the other side, these oligo- and polymers possess a good to very good solubility, especially in common organic solvents. Therefore the oligo- and polymers according to the invention are amenable to fast and economically favorable solution processing.

A further aspect of the invention relates to reactive mesogens consisting of a central core comprising one or more 3-(1,1-difluoroalkyl)thiophene units, and optionally comprising further conjugated moieties that form an extended conjugated system together with the 3-(1,1-difluoro-alkyl)thiophene units, said core being linked, optionally via a spacer group, to one or two polymerisable groups. The reactive mesogens can induce or enhance liquid crystal phases or are liquid crystalline themselves. They can be ordered and aligned in their mesophase and the polymerisable group can be polymerised or crosslinked in situ to form coherent polymer films with a high degree of long range order, or monodomain, thus yielding improved semiconductor materials with high stability and high charge carrier mobility.

A further aspect of the invention relates to liquid crystal polymers, in particular liquid crystal side chain polymers obtained from the reactive mesogens according to the present invention, which are then further processed e.g. from solution as thin layers for use in semiconductor devices.

A further aspect of the invention relates to the mono-, oligo- and polymers, a material or polymer film according to the invention, which are oxidatively or reductively doped to form conducting ionic species. Another aspect of the invention is a charge injection layer, planarising layer, antistatic film or conducting substrate or pattern for electronic applications or flat panel displays, comprising mono-, oligo- or polymers, a material or polymer film according to this invention.

### Definition of Terms

The terms 'liquid crystalline or mesogenic material' or 'liquid crystalline or mesogenic compound' means materials or compounds comprising one or more rod-shaped, lath-shaped or disk-shaped mesogenic groups, i.e. groups with the ability to induce liquid crystal phase behaviour. The compounds or materials comprising mesogenic groups do not necessarily have to exhibit a liquid crystal phase themselves. It is also possible that they show liquid crystal phase behaviour only in mixtures with other compounds, or when the mesogenic compounds or materials, or the mixtures thereof, are polymerised.

The term 'polymerisable' includes compounds or groups that are capable of participating in a polymerisation reaction, like radicalic or ionic chain polymerisation, polyaddition or polycondensation, and reactive compounds or reactive groups that are capable of being grafted for example by condensation or addition to a polymer backbone in a polymeranaloguous reaction.

The term 'film' includes self-supporting, i.e. free-standing, films that show more or less pronounced mechanical stability and flexibility, as well as coatings or layers on a supporting substrate or between two substrates.

### Summary of the Invention

The invention relates to mono-, oligo- and polymers comprising at least one 3-(1,1-difluoro-alkyl)thiophene group, as claimed in claim 1.

The invention further relates to the use of mono-, oligo- and polymers according to the invention as semiconductors or charge transport materials, in particular in optical, electrooptical or electronic devices, like for example in field effect transistors (FET) as components of integrated circuitry, as thin film transistors in flat panel display applications or for Radio Frequency Identification (RFID) tags, or in semiconducting components for organic light emitting diode (OLED) applications such as electroluminescent displays or backlights of e.g. liquid crystal displays, for photovoltaic or sensor devices, as electrode materials in batteries, as photoconductors and for electrophotographic applications like electrophotographic recording.

The invention further relates to a field effect transistor, for example as a component of integrated circuitry, as a thin film transistor in flat panel display applications, or in a Radio Frequency Identification (RFID) tag, comprising one or more mono-, oligo- or polymers according to the invention.

The invention further relates to a semiconducting component, for example in OLED applications like electroluminescent displays or backlights of e.g. liquid crystal displays, in photovoltaic or sensor devices, as electrode materials in batteries, as photoconductors and for electrophotographic applications, comprising one or more mono-, oligo- or polymers according to the invention.

The invention further relates to a security marking or device comprising an RFID or ID tag or a FET according to the invention.

### Detailed Description of the Invention

The mono-, oligo- and poly-[3-(1,1-difluoroalkyl)thiophenes] according to the invention comprise one or more identical or different recurring units of formula I

-[(Y)ₐ-(D)_{b}-(Z)_{c}]- I

wherein
- D: is a 3-(1,1-difluoroalkyl)thiophene group of formula II

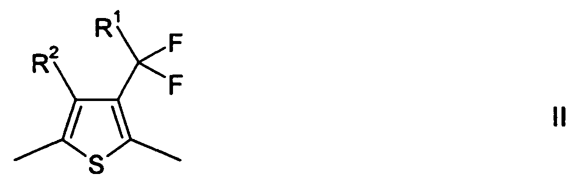

- R¹: is H, straight chain, branched or cyclic alkyl with 1 to 20 C-atoms, which may be unsubstituted, mono- or partially-substituted by F, Cl, Br, I, -CN or -OH, it being also possible for one or more non-adjacent CH₂ groups to be replaced, in each case independently from one another, by -O-, -S-, -NH-, -NR⁰-, -SiR⁰R⁰⁰-, -CO-, -COO- , -OCO-, -OCO-O-, -S-CO-, -CO-S-, -CH=CH- or -C≡C- in such a manner that O and/or S atoms are not linked directly to one another, or optionally substituted aryl or heteroaryl,
- R²: is H, F, straight chain, branched or cyclic alkyl with 1 to 20 C-atoms, which may be unsubstituted, mono- or poly- substituted by F, Cl, Br, I, -CN or -OH, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -CO-, -CO-S-, -CH=CH- or -C≡C- in such a manner that O and/or S atoms are not linked directly to one another, or optionally substituted aryl or heteroaryl,
- R° and R⁰⁰: are independently of each other H or alkyl with 1 to 12 C-atoms,
- Y and Z: are independently of each other -CX¹=CX²-, -C≡C-, or optionally substituted arylene or heteroarylene,

- X¹ and X²: are independently of each other H, F, Cl or CN, and
- a, b and c: are independently of each other 0 or 1, with a + b + c > 0, and wherein in at least one recurring unit b is 1.

In the definition of R¹ "partially substituted" means that not all H-atoms are substituted by the given substituants, especially R¹ is not perfluorinated or perchlorinated.

The mono-, oligo- and poly-3-(1,1-difluoro-alkyl)thiophenes according to the invention are especially useful as charge transport semiconductors in that they have high carrier mobilities.

Particularly preferred are those mono-, oligo- and polymers wherein R¹ is an alkyl-group with 1 to 20 C-atoms, especially with 2 to 15 C-atoms, which may contain one CF₂- or CF₃-group, without being perfluorinated.

Additionally those mono-, oligo- and polymers according to the invention are preferred wherein R² is H, F or an optionally substituted alkyl-group with 1 to 20 C-atoms.

The mono-, oligo- and polymers comprise at least one 3-(1,1-difluoro-alkyl)thiophene group and at least one reactive group that is capable of a polymerisation or crosslinking reaction.

Further preferred are mono-, oligo- and polymers comprising at least one 3-(1,1-difluoro-alkyl)thiophene group that are mesogenic or liquid crystalline.

Further preferred are oligo- and polymers comprising at least two recurring units, at least one of which is a recurring unit according to the invention.

The mono-, oligo- and polymers are selected of formula I1

R⁵-[(Y)ₐ-(D)_{b}-(Z)_{c}]ₙ-R⁶ l1

wherein Y, Z, D, a, b and c are as defined in formula I,
- n: is an integer from 1 to 5000,
- R⁵ and R⁶: are independently of each other H, halogen, Sn(R⁰)₃ or straight chain, branched or cyclic alkyl with 1 to 20 C- atoms, which may be unsubstituted, mono- or poly- substituted by F, Cl, Br, I or CN, it being also possible for one or more non-adjacent CH₂ groups to be replaced, in each case independently from one another, by -O-, -S-, -NH-, -NR°-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, or optionally substituted aryl or heteroaryl, or denote P-Sp-X,
- P: is a polymerisable or reactive group,
- Sp: is a spacer group or a single bond, and
- X: is -O-, -S-, -OCH₂-, -CH₂O-, -CO-, -COO-, -OCO-, -OCO-O-, -CO-NR⁰-, -NR⁰-CO-, -OCH₂-, -CH₂O-, -SCH₂- , -CH₂S-, -CH=CH-COO-, -OOC-CH=CH- or a single bond,
wherein R° and R⁰⁰ are as defined above and wherein the recurring units [(Y)ₐ-(D)_{b}-(Z)_{c}] can be identical or different, characterized in that one or both of R⁵ and R⁶denote P-Sp-X.

In the oligo- and polymers of the present invention the recurring units (Y)ₐ-(D)_{b}-(Z)_{c} in case of multiple occurrence can be selected of formula I independently of each other, so that an oligo- or polymer may comprise identical or different recurring units (Y)ₐ-(D)_{b}-(Z)_{c}. The oligo- and polymers thus include homopolymers and copolymers like for example
- statistically random copolymers, for example with a monomer sequence such as -Y-D-Z-Z-D-Y-D-,
- alternating copolymers, for example with a monomer sequence such as -Y-D-Z-Y-D-Z-, and
- block copolymers, for example with a monomer sequence such as -Y-Y-D-D-D-Z-Z-Z-Z-,
   wherein the groups Y and Z form a conjugated system together with the 3-(1,1-difluoro-alkyl)thiophene unit D.

Especially preferred is the homopolymer.

Further preferred are mono-, oligo- and polymers comprising one or more recurring units (Y)ₐ-(D)_{b}-(Z)_{c}, wherein a = c = 0 and b = 1, very preferably consisting exclusively of such recurring units.

Further preferred are mono-, oligo- and polymers comprising one or more recurring units (Y)ₐ-(D)_{b}-(Z)_{c}, wherein b = c = 1 and a = 0, very preferably consisting exclusively of such recurring units.

Further preferred are mono-, oligo- and polymers comprising one or more recurring units (Y)ₐ-(D)_{b}-(Z)_{c}, wherein a = b = c = 1, very preferably consisting exclusively of such recurring units.

Further preferred are mono-, oligo- and polymers wherein
- n is an integer greater than 1,
- n is an integer from 2 to 5000, in particular from 30 to 1000,
- n is an integer from 2 to 5,
- n is an integer from 1 to 15 and one or both of R⁵ and R⁶ denote P-Sp-X,
- n is an integer from 2 to 5000 and R⁵ and R⁶ have one of the meanings of R²,
- the molecular weight is from 30000 to 300000,
- R¹ is selected from C₁-C₂₀-alkyl that is optionally substituted with one or more fluorine atoms, but not perfluorinated, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, and optionally substituted aryl or heteroaryl,
- R¹ is a C₁-C₂₀-alkyl group,
- R² is selected from H, F, C₁-C₂₀-alkyl that is optionally substituted with one or more fluorine atoms, very preferably not perfluorinated, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, and optionally substituted aryl or heteroaryl,
- R² is selected from H, F or alkyl with 1 to 20 C-atoms, which is optionally substituted with one or more fluorine atoms, very preferably not perfluorinated,
- R⁵ and R⁶ are selected from H, halogen, C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, C₁-C₂₀-fluoroalkyl, and optionally substituted aryl or heteroaryl, in particular from H, halogen, C₁-C₂₀-alkyl and C₁-C₂₀-alkoxy,
- Y and Z are optionally substituted arylene or heteroarylene,
- Y and Z are -CX¹=CX²- or -C≡C-,
- in at least one monomer unit (Y)ₐ-(D)_{b}-(Z)_{c} a, b and c are 1, and one of Y and Z is arylene or heteroarylene and the other is -CX¹=CX²- or -C≡C-,
- -CX¹=CX²- is -CH=CH-, -CH=CF-, -CF=CH-, -CF=CF-, -CH=C(CN)- or -C(CN)=CH-,
- if n = b = 1 and a = c = 0, at least one of R⁵ and R⁶ is different from H.

A further preferred embodiment of the present invention relates to mono-, oligo- and polymers that are mesogenic or liquid crystalline, in particular those comprising one or more polymerisable groups. Very preferred materials of this type are monomers and oligomers of formula 11 wherein n is an integer from 1 to 15 and R⁵ and/or R⁶ denote P-Sp-X.

These materials are particularly useful as semiconductors or charge transport materials, as they can be aligned into uniform highly ordered orientation in their liquid crystal phase by known techniques, thus exhibiting a higher degree of order that leads to particularly high charge carrier mobility. The highly ordered liquid crystal state can be fixed by in situ polymerisation or crosslinking via the groups P to yield polymer films with high charge carrier mobility and high thermal, mechanical and chemical stability.

It is also possible to copolymerise the polymerisable mono-, oligo-and polymers according to the invention with other polymerisable mesogenic or liquid crystal monomers that are known from prior art, in order to induce or enhance liquid crystal phase behaviour.

Thus, another aspect of the invention relates to a polymerisable liquid crystal material comprising one or more mono-, oligo- or polymers according to the invention comprising at least one polymerisable group, and optionally comprising one or more further polymerisable compounds, wherein at least one of the polymerisable mono-, oligo- and poly-3-(1,1-difluoroalkyl)thiophenes and/ or the further polymerisable compounds is mesogenic or liquid crystalline.

Particularly preferred are liquid crystal materials having a nematic and/or smectic phase. For FET applications smectic materials are especially preferred. For OLED applications nematic or smectic materials are especially preferred.

Another aspect of the present invention relates to an anisotropic polymer film with charge transport properties obtainable from a polymerisable liquid crystal material as defined above that is aligned in its liquid crystal phase into macroscopically uniform orientation and polymerised or crosslinked to fix the oriented state.

Another aspect of the invention relates to a liquid crystal side chain polymer (SCLCP) obtained from a polymerisable liquid crystal material as defined above by polymerisation or polymeranaloguous reaction. Particularly preferred are SCLCPs obtained from one or more monomers according to formula 11 wherein one or both of R⁵ and R⁶ are a polymerisable or reactive group, or from a polymerisable mixture comprising one or more of such monomers of formula 11.

Another aspect of the invention relates to an SCLCP obtained from one or more monomers of formula 11 wherein one or both of R⁵ and R⁶ are a polymerisable group, or from a polymerisable liquid crystal mixture as defined above, by copolymerisation or polymeranaloguous reaction together with one or more additional mesogenic or non-mesogenic comonomers.

Side chain liquid crystal polymers or copolymers (SCLCPs), in which the semiconducting component is located as a pendant group, separated from a flexible backbone by an aliphatic spacer group, offer the possibility to obtain a highly ordered lamellar like morphology. This structure consists of closely packed conjugated aromatic mesogens, in which very close (typically < 4 Å) pi-pi stacking can occur. This stacking allows intermolecular charge transport to occur more easily, leading to high charge carrier mobilities. SCLCPs are advantageous for specific applications as they can be readily synthesized before processing and then e.g. be processed from solution in an organic solvent. If SCLCPs are used in solutions, they can orient spontaneously when coated onto an appropriate surface and when at their mesophase temperature, which can result in large area, highly ordered domains.

Especially preferred are mono-, oligo- and polymers of the following formulae 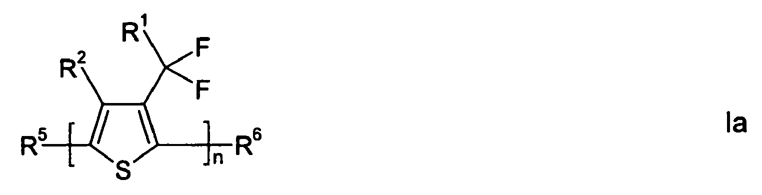 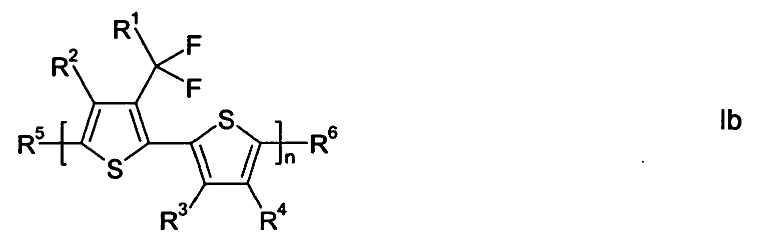 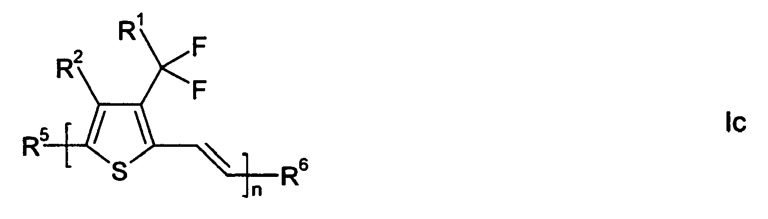 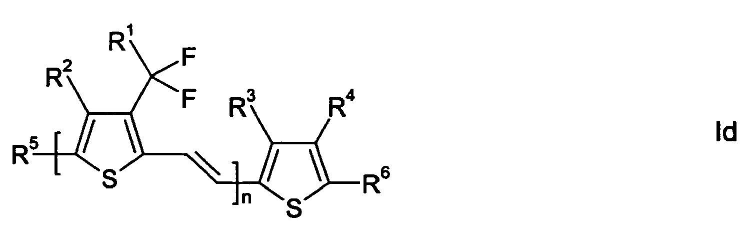 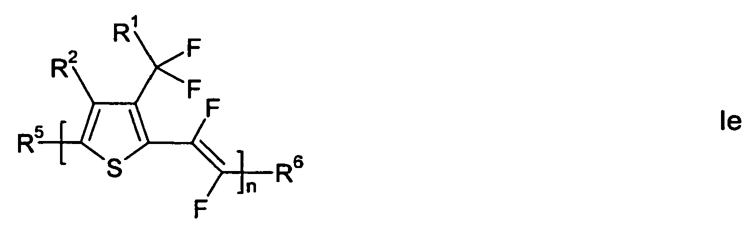 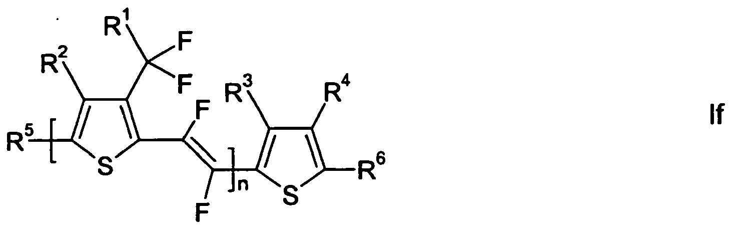 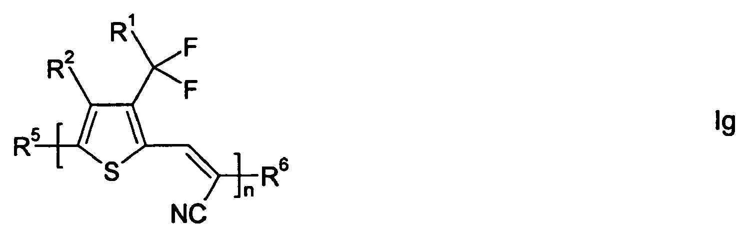 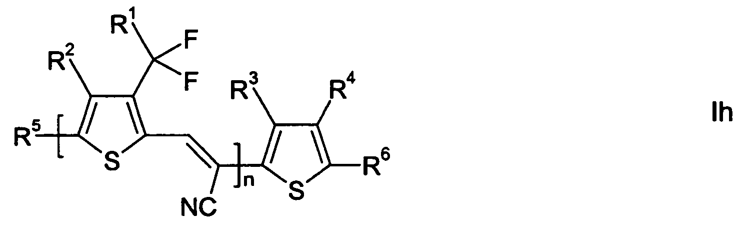 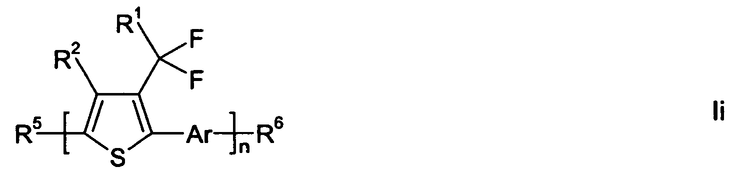 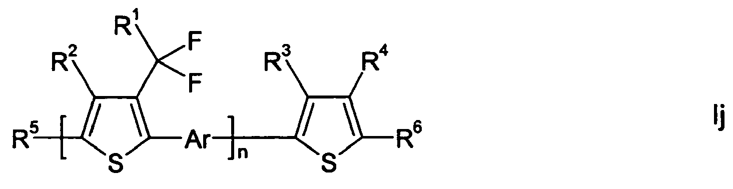 wherein
R¹ and R² have the meanings given in formula II,
R⁵, R⁶ and n have the meanings given in formula 11,
R³, R⁴ independently of each other have one of the meanings of R² given in formula II,
   - Ar: is a bivalent mono-, bi- or tricyclic aromatic or heteroaromatic group with up to 25 C atoms that may also comprise condensed rings and is optionally mono- or poly-substituted with F, Cl and/ or groups R² as defined in formula II.

In these preferred formulae, R² to R⁵ are very preferably H, F or alkyl with 1-16 C atoms that is optionally fluorinated, and Ar is very preferably 1,4-phenylene, alkoxyphenylene, alkylfluorene, thiophene-2,5-diyl, thienothiophene-2,5-diyl or dithienothiophene-2,6-diyl, all which may be substituted as defined for Ar. R¹ is preferably alkyl with 1-20 C-atoms, especially with 2-15 C-atoms. R³, R⁴ have preferably the meanings of R² and/ or -CF₂-R¹. Very preferably R³ has the same meaning as R² and R⁴ is -CF₂-R¹.

Aryl and heteroaryl preferably denote a mono-, bi- or tricyclic aromatic or heteroaromatic group with up to 25 C atoms that may also comprise condensed rings and is optionally substituted with one or more groups R².

Especially preferred aryl and heteroaryl groups are phenyl in which, in addition, one or more CH groups may be replaced by N, naphthalene, thiophene, thienothiophene, dithienothiophene, fluorene and oxazole, all of which can be unsubstituted, mono- or polysubstituted with L, wherein L is halogen or an alkyl, alkoxy, alkylcarbonyl or alkoxycarbonyl group with 1 to 12 C atoms, wherein one or more H atoms may be replaced by F or Cl.

If in the formulae shown above and below one of R¹ to R⁶ is an alkyl or alkoxy radical, i.e. where the terminal CH₂ group is replaced by -O-, this may be straight-chain or branched. It is preferably straight-chain, has 2 to 8 carbon atoms and accordingly is preferably ethyl, propyl, butyl, pentyl, hexyl, heptyl, octyl, ethoxy, propoxy, butoxy, pentoxy, hexyloxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Especially preferred meanings of R¹ are linear alkyl chains of the formula -C_{q}H_{2q+1}, wherein q is an integer from 2 to 15.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Fluoroalkyl is preferably CᵢF₂ᵢ₊₁, wherein i is an integer from 1 to 15, in particular CF₃, C₂F₅, C₃F₇, C₄F₉, C₅F₁₁, C₆F₁₃, C₇F₁₅ or C₈F₁₇, very preferably C₆F₁₃.

Halogen is preferably F or Cl.

The polymerisable or reactive group P is selected from CH₂=CW¹-COO-, 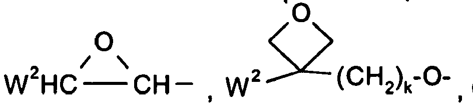 CH₂=CW²-(O)ₖ₁-, CH₃-CH=CH-O-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN- and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

Especially preferred groups P are CH₂=CH-COO-, CH₂=C(CH₃)-COO-, CH₂=CH-, CH₂=CH-O- and 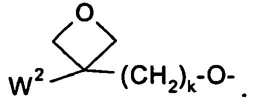 Very preferred are acrylate and oxetane groups. Oxetanes produce less shrinkage upon polymerisation (cross-linking), which results in less stress development within films, leading to higher retention of ordering and fewer defects. Oxetane cross-linking also requires cationic initiator, which unlike free radical initiator is inert to oxygen.

As for the spacer group Sp all groups can be used that are known for this purpose to those skilled in the art. The spacer group Sp is preferably a linear or branched alkylene group having 1 to 20 C atoms, in particular 1 to 12 C atoms, in which, in addition, one or more non-adjacent CH₂ groups may be replaced by -O-, -S-, -NH-, -N(CH₃)-, -CO-, -O-CO-, -S-CO-, -O-COO-, -CO-S-, -CO-O-, -CH(halogen)-, -C(halogen)₂, -CH(CN)-, -CH=CH- or -C≡C-, or a siloxane group.

Typical spacer groups are for example -(CH₂)ₚ-, -(CH₂CH₂O)ᵣ-CH₂CH₂-, -CH₂CH₂-S-CH₂CH₂- or -CH₂CH₂-NH-CH₂CH₂- or -(SiR⁰R⁰⁰-O)ₚ-, with p being an integer from 2 to 12, r being an integer from 1 to 3 and R⁰ and R⁰⁰ having the meanings given in formula I.

Preferred spacer groups are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyliminoethylene, 1-methylalkylene, ethenylene, propenylene and butenylene for example.

Further preferred are compounds with one or two groups P-Sp-X
wherein Sp and/or X is a single bond.

In case of compounds with two groups P-Sp-X, each of the two polymerisable groups P, the two spacer groups Sp, and the two linkage groups X can be identical or different.

SCLCPs obtained from the inventive compounds or mixtures by polymerisation or copolymerisation have a backbone that is formed by the polymerisable group P in formula 11.

The mono-, oligo- and polymers of the present invention can be synthesized according to or in analogy to known methods. Some preferred methods are described below.

### Regioregular head-to-tail poly[3-(1,1-difluoroalkyl)thiophenes] (4):

Starting from commercially available 3-cyanothiophene (**5**), a route to poly[3-(1,1-difluoroalkyl)thiophenes] (**4**) is outlined below. 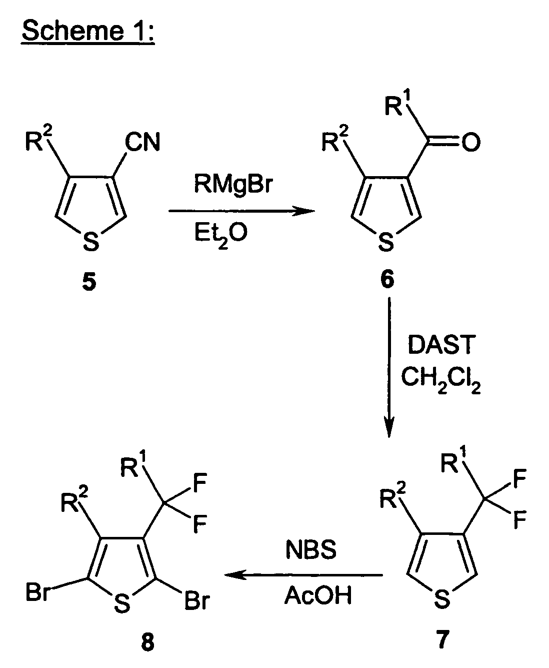 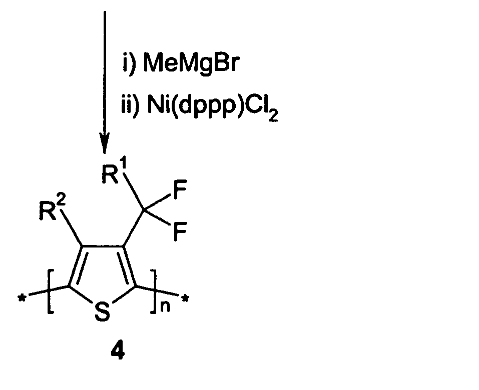 3-Cyanothiophene (**5**) is alkylated using the alkyl Grignard to yield ketothiophene (**6**), via hydrolysis of the intermediate imine. Ketothiophene (**6**) is converted into a thiolane (**70** by treatment with a dithiol in the presence of boron trifluoride.Fluorination of thiolane (**7**) using NBS in pyridinium poly(hydrogen fluoride) afforded 1,1-difluoroalkylthiophene (**7**) as a mixture containing the mono- and dibromothiophenes (see reference 9). The crude mixture was further brominated with NBS in DMF to afford dibromo-difluoroalkylthiophene (**8**) after distillation. Poly[3-(1,1-difluoroalkyl)-thiophene] (**4**) is synthesised from (**8**) by the route described by Rieke [see reference 10]. Treatment with one equivalent of active zinc forms the arylzinc reagent. Addition of a catalytic amount of Ni(dmpe)Cl_{2,} where dppp is 1,2- bis(dimethylphosphino)ethane, affords regioregular head-to-tail poly[3-(1,1-difluoroalkyl)thiophene] (**4**).

Two alternative strategies for forming the key 1,1-difluoroalkylthiophene (**7**) intermediate are outlined in scheme 2.

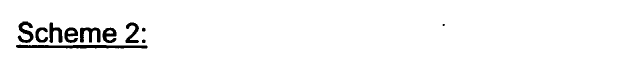

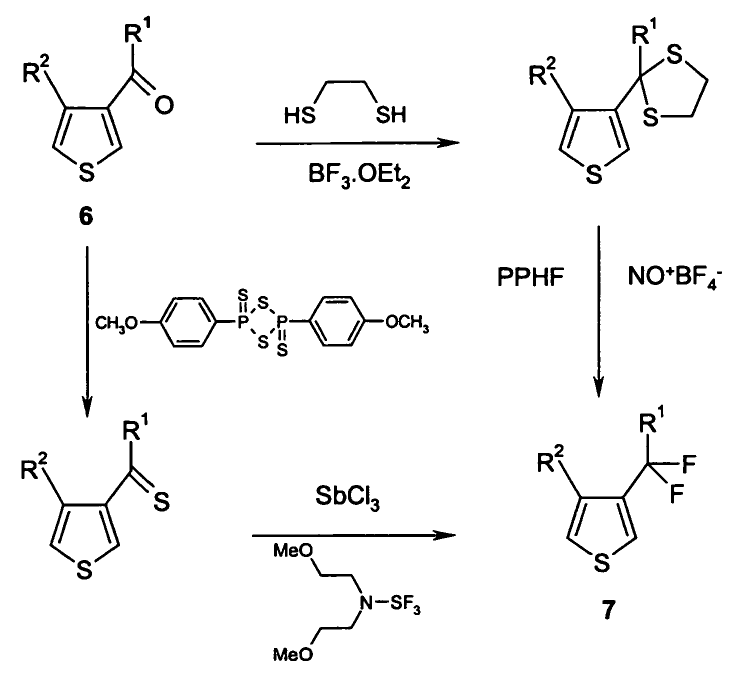

The ketothiophene (**6**) intermediate is converted into a thioketone by reaction with Lawessons reagent (2,4-bis(4-methoxyphenyl)-1,3,2,4-dithiadiphosphetan- 2,4-dithione). The thioketone is converted into (**7**) by treatment with bis(2-methoxyethyl)aminosulfur trifluoride [see reference 11]. Alternatively intermediate (**6**) is converted directly into 1,1-difluoroalkylthiophene (**7**) using (diethylamino)sulfur trifluoride (DAST) or equivalent [see reference 12].

Other coupling routes to polymer (**7**) are Stille coupling [see reference 13], McCullough coupling [see reference 14, 15], and Suzuki coupling [see reference 16].

Reactive mesogens comprising an 3-(1,1-difluoro-alkyl)thiophene mono- or oligomeric group and one or two polymerisable groups P-Sp-X can be prepared for example according to or in analogy to the following synthesis routes.

According to scheme 3, 3-(1,1-difluoro-alkyl)thiophene (**7**) is brominated in the 2-position by one equivalent of N-bromo-succinimide in acetic acid to yield (**9**). Cross-coupling of the dihalo 3-(1,1-difluoro-alkyl)thiophene (**9**) with an alkyl Grignard reagent in the presence of a nickel catalyst yields mono-alkyl alcohol (**10**). Lithiation of (**10**) in the 5-position, followed by oxidative coupling affords (**11**). Routine methodology converts the protected bis-alcohol (**11**) into the bis-acrylate or bis-oxetane. 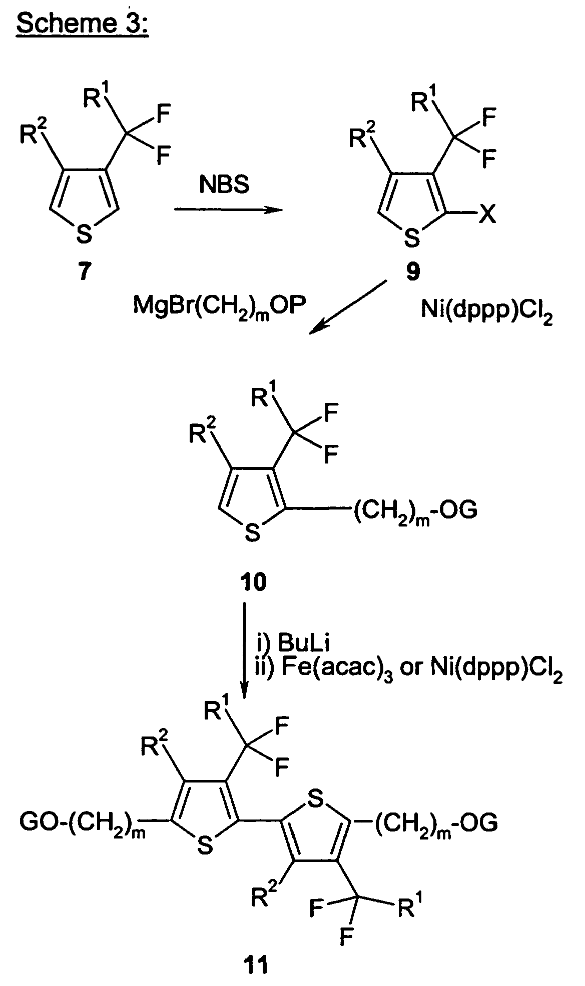 wherein m is an integer e.g. from 1 to 20 and G is a protecting group.

### 3-(1,1-difluoro-alkyl)thiophene polymers containing conjugated groups CX¹=CX² or Ar

The Stille coupling of 2,5-dibromo-3-(1,1-difluoro-alkyl)thiophene (**8**) with the bis-organotin reagent (**12**) yields polymer (**13**) containing CX¹=CX² groups [see reference 17].

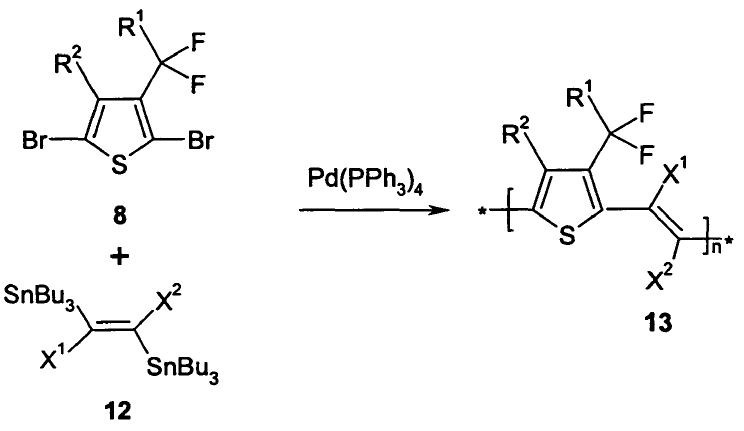

The Suzuki coupling of 2,5-dibromo-3-(1,1-difluoro-alkyl)thiophene (**8**) with bis-boronic acid (**14**) yields polymer (**15**) containing aryl groups.

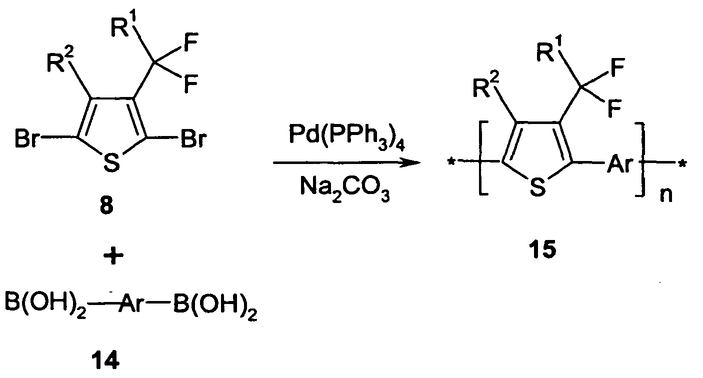

A further aspect of the invention relates to both the oxidised and reduced form of the compounds and materials according to this invention. Either loss or gain of electrons results in formation of a highly delocalised ionic form, which is of high conductivity. This can occur on exposure to common dopants. Suitable dopants and methods of doping are known to those skilled in the art, e.g. from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g. I₂, Cl₂, Br₂, ICI, ICl₃, IBr and IF), Lewis acids (e.g. PF₅, AsF₅, SbF₅, BF₃, BCl₃, SbCl₅, BBr₃ and SO₃), protonic acids, organic acids, or amino acids (e.g. HF, HCI, HNO₃, H₂SO₄, HClO₄, FSO₃H and CISO₃H), transition metal compounds (e.g. FeCl₃, FeOCI, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g. Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g. H⁺, Li⁺, Na⁺, K⁺, Rb⁺ and Cs⁺), alkali metals (e.g., Li, Na, K, Rb, and Cs), alkaline-earth metals (e.g., Ca, Sr, and Br), O₂, XeOF₄, (NO₂⁺) (SbF₆⁻), (NO₂⁺) (SbCl₆⁻), (NO₂⁺) (BF₄⁻), AgClO₄, H₂IrCl₆, La(NO₃)₃ 6H₂O, FSO₂OOSO₂F, Eu, acetylcholine, R₄N⁺, (R is an alkyl group), R₄P⁺ (R is an alkyl group), R₆As⁺ (R is an alkyl group), and R₃S⁺ (R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns of tracts in electronic applications such as printed circuit boards and condensers.

Mono-, oligo- and polymers according to the present invention that comprise one or more groups P-Sp-X can be polymerised, or copolymerised with other polymerisable compounds, via the polymerisable group P. This is preferably done by in-situ polymerisation of a coated layer of the material, preferably during fabrication of the electronic or optical device comprising the inventive semiconductor material. In case of liquid crystal materials, these are preferably aligned in their liquid crystal state into homeotropic orientation prior to polymerisation, where the conjugated pi-electron systems are orthogonal to the direction of charge transport. This ensures that the intermolecular distances are minimised and hence then energy required to transport charge between molecules is minimised. The molecules are then polymerised or crosslinked to fix the uniform orientation of the liquid crystal state. Alignment and curing are carried out in the liquid crystal phase or mesophase of the material. This technique is known in the art and is generally described for example in D.J. Broer, et al., Angew. Makromol. Chem. 183, (1990), 45-66

Alignment of the liquid crystal material can be achieved for example by treatment of the substrate onto which the material is coated, by shearing the material during or after coating, by application of a magnetic or electric field to the coated material, or by the addition of surface-active compounds to the liquid crystal material. Reviews of alignment techniques are given for example by I. Sage in "Thermotropic Liquid Crystals", edited by G. W. Gray, John Wiley & Sons, 1987, pages 75-77, and by T. Uchida and H. Seki in "Liquid Crystals - Applications and Uses Vol. 3", edited by B. Bahadur, World Scientific Publishing, Singapore 1992, pages 1-63. A review of alignment materials and techniques is given by J. Cognard, Mol. Cryst. Liq. Cryst. 78, Supplement 1 (1981), pages 1-77.

Polymerisation takes place by exposure to heat or actinic radiation. Actinic radiation means irradiation with light, like UV light, IR light or visible light, irradiation with X-rays or gamma rays or irradiation with high energy particles, such as ions or electrons. Preferably polymerisation is carried out by UV irradiation at a non-absorbing wavelength. As a source for actinic radiation for example a single UV lamp or a set of UV lamps can be used. When using a high lamp power the curing time can be reduced. Another possible source for actinic radiation is a laser, like e.g. a UV laser, an IR laser or a visible laser.

Polymerisation is preferably carried out in the presence of an initiator absorbing at the wavelength of the actinic radiation. For example, when polymerising by means of UV light, a photoinitiator can be used that decomposes under UV irradiation to produce free radicals or ions that start the polymerisation reaction. When curing polymerisable materials with acrylate or methacrylate groups, preferably a radical photoinitiator is used, when curing polymerisable materials with vinyl, epoxide and oxetane groups, preferably a cationic photoinitiator is used. It is also possible to use a polymerisation initiator that decomposes when heated to produce free radicals or ions that start the polymerisation. As a photoinitiator for radical polymerisation for example the commercially available Irgacure 651, Irgacure 184, Darocure 1173 or Darocure 4205 (all from Ciba Geigy AG) can be used, whereas in case of cationic photopolymerisation the commercially available UVI 6974 (Union Carbide) can be used.

The polymerisable material can additionally comprise one or more other suitable components such as, for example, catalysts, sensitizers, stabilizers, inhibitors, chain-transfer agents, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

Mono-, oligo- and polymers comprising one or more groups P-Sp-X can also be copolymerised with polymerisable mesogenic compounds to induce, or, in case of mesogenic materials of formula I, enhance liquid crystal phase behaviour. Polymerisable mesogenic compounds that are suitable as comonomers are known in prior art and disclosed for example in WO 93/22397; EP 0,261,712; DE 195,04,224; WO 95/22586 and WO 97/00600.

SCLCPs can be prepared from the polymerisable compounds or mixtures according to the invention by the methods described above, or by conventional polymerisation techniques which are known to those skilled in the art, including for example radicalic, anionic or cationic chain polymerisation, polyaddition or polycondensation. Polymerisation can be carried out for example as polymerisation in solution, without the need of coating and prior alignment, or polymerisation in situ. It is also possible to form SCLCPs by grafting compounds according to the invention with a suitable reactive group, or mixtures thereof, to presynthesized isotropic or anisotropic polymer backbones in a polymeranaloguous reaction. For example, compounds with a terminal hydroxy group can be attached to polymer backbones with lateral carboxylic acid or ester groups, compounds with terminal isocyanate groups can be added to backbones with free hydroxy groups, compounds with terminal vinyl or vinyloxy groups can be added e.g. to polysiloxane backbones with Si-H groups. It is also possible to form SCLCPs by copolymerisation or polymeranaloguous reaction from the inventive compounds together with conventional mesogenic or non mesogenic comonomers. Suitable comonomers are known to those skilled in the art. In principle it is possible to use all conventional comonomers known in the art that carry a reactive or polymerisable group capable of undergoing the desired polymer-forming reaction, like for example a polymerisable or reactive group P as defined above. Typical mesogenic comonomers are for example those mentioned in WO 93/22397; EP 0,261,712; DE 195,04,224; WO 95/22586 and WO 97/00600. Typical non mesogenic comonomers are for example alkyl mono- or diacrylates or alkyl mono- or dimethacrylates with alkyl groups of 1 to 20 C atoms, like methyl acrylate or methyl methacrylate, trimethylpropane trimethacrylate or pentaerythritol tetraacrylate.

Especially the oligomers and polymers according to the invention show advantageous solubility properties which allow production processes using solutions of these compounds. Thus films, including layers and coatings, may be generated by low cost production techniques e.g. spin coating. Suitable solvents or solvent mixtures comprise alkanes and/ or aromatics, especially their fluorinated derivatives. Preferred solvents are propylene glycol monoethyl acetate, methoxy propanol, ethyl lactate, cyclohexanone and cyclopropanone and mixtures comprising one or more of these solvents.

The mono-, oligo- and poly-3-(1,1-difluoro-alkyl)thiophenes of the present invention are useful as optical, electronic and semiconductor materials, in particular as charge transport materials in field effect transistors (FETs), as photovoltaics or sensor materials, for electrophotographic applications, and for other semiconductor applications. Such FETs, where an organic semiconductive material is arranged as a film between a gate-dielectric and a drain and a source electrode, are generally known, e.g. US 5,892,244, WO 00/79617, US 5,998,804, also see references 1, 18 and 19. The solubility properties of these materials according to the invention, allow amenability to solution processing, and therefore low cost, high volume manufacture by techniques such as reel to reel coating is possible. Preferred applications of these FETs are therefore such as integrated circuitry, TFT-displays and security applications. In security applications, field effect transistors and other devices with semiconductive materials, like transistors or diodes, may be used for ID tags or security markings to authenticate and prevent counterfeiting of documents of value like banknotes, credit cards or ID cards, national ID documents, licenses or any product with money value, like stamps, tickets, shares, cheques etc..

Alternatively, the mono-, oligo- and polymers according to the invention may be used in organic light emitting devices or diodes (OLEDs), e. g. in display applications or as backlight of e.g. liquid crystal displays. Common OLEDs are realized using multilayer structures. An emission layer is generally sandwiched between one or more electron-transport and/ or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/or in the emission layer, corresponding to their electrical and/ or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see e. g. Meerholz, Synthetic Materials, 111-112, 2000, 31-34, Alcala, J. Appl. Phys., 88, 2000, 7124-7128 and the literature cited therein.

According to another use, the inventive compounds, materials or films, especially those which show photoluminescent properties, may be employed as materials of light sources, e.g. of display devices such as described in EP 0 889 350 A1 or by C. Weder et al., Science, 279, 1998, 835-837.

### References:

1. H. E. Katz, Z. Bao and S. L. Gilat, Acc. Chem. Res., 2001, 34, 5, 359.
2. S.F. Nelson, Y.Y. Lin, D.J. Gundlach and T.N. Jackson, Appl. Phys. Lett., 1998, 72, 1854.
3. Z. Bao, A. Dodabalapur and A. J. Lovinger, Appl. Phys. Lett., 1996, 69, 4108.
4. H. Sirringhaus, N. Tessler, D. S. Thomas, P. J. Brown, R. H. Friend, Adv. Solid State Phys., 1999, 39,101.
5. L. Robitaille and M. Leclerc, Macromol. 1994, 27, 1847-1851.
6. Y. Sakamoto, et al., J. Am. Chem. Soc., 2001, 123, 4643-4644.
7. A.Facchetti, et al., Angew. Chem. Int. Ed., 2000, 39, 4547-4551.
8. EP 0 414 906 A1.
9. C. York, et al., Tetrahedron, 1996, 52, 9-14.
10. T.-A. Chen, R. D. Rieke, J. Am. Chem. Soc., 1992, 114, 10087.
11. G. S. Lal, A. Evans, J. Org. Chem., 2000, 65, 4830-4832.
12. W.J. Middelton, J. Org. Chem., 1975, 40, 574.
13. D. Milstein, J. K. Stille, J. Am. Chem. Soc., 1979, 101, 4992.
14 Loewe, R.S., S.M. Khersonsky, and R.D. McCullough, Advanced Materials, 1999. 11(3), 250-253.
15. Loewe, R.S., et al., Macromolecules, 2001, 34, 4324-4337.
16. N. Miyaura, T. Yanagi, A. Suzuki, Synth. Commun., 1981, 11, 513.
17. R. S. Loewe and R. D. McCullough, Chem. Mater., 2000, 12, 3214.
18. H. Fuchigami, A. Tsumura, H. Koezuka, Appl. Phys. Lett., 1993, 63, 1372-1374.
19. H. Sirringhaus, N. Tessler, R. H. Friend, Science, 1998, 280, 1741-1744.

### Examples

### Reference Example 1

### 3-(1'-hexanoyl)thiophene (6)

3-Cyanothiophene (20.0g, 0.183 mol) was dissolved in anhydrous diethyl ether (100 ml) and cooled to 0°C. A solution of pentylmagnesium bromide (2M in diethylether, 100 ml) was added dropwise via a dropping funnel. The reaction was allowed to warm to RT and stirred for 16h. The reaction was quenched with 5% HCI (150 ml) and stirred for 1 h. The organic layer was separated and the aqueous layer extracted with THF (3 x 100 ml). The combined organics were washed with brine, dried (Na₂SO₄), filtered and concentrated under reduced pressure to afford a dark oil (33.14 g, 99%). Purity (HPLC) = 97%. MS (El) M = 182 (M⁺). The ¹H NMR was consistent with the proposed structure.

### Reference Example 2

### Thiolane protected thiophene 7

3-(1'-hexanoyl)thiophene (33.14g, 0.182 mol) and ethanedithiol(32 ml, 0.382 mol) were stirred under nitrogen and boron trifluoride acetic acid complex ((27.8 ml, 0.2 mol) was added dropwise. The reaction was stirred for 16 h at RT. Further dithiol (5 ml) and BF₃.HOAc (5 ml) were added and the reaction stirred for a further 24 h. The reaction was diluted with petrol (200 ml) and quenched with sat. NaHCO₃ (100 ml). The layers were separated and the organic layer was washed with 5% NaOH (3 x 50 ml), brine, dried (Na₂SO₄), filtered and concentrated under reduced pressure to afford a crude oil (44g). This was further purified by column chromatography (eluent petrol, then petrol:DCM 7:3) to afford **7** (30.9 g, 66%). Purity (HPLC) =99%. MS (El) M = 258 (M⁺). The ¹H NMR was consistent with the proposed structure.

### Reference Example 3

### 2,5-dibromo-3-(1',1'-difluorohexyl)thiophene (8)

Recrystallised NBS (45.45g, 255 mmol) was dissolved in anhydrous DCM (350 ml) in a PFA flask and cooled to -78C. 70% Pyridinium poly(hydrogen fluoride) (PPHF, 30 ml) was added via a plastic syringe, followed by the dropwise addition of thiolane (**7**). The reaction was stirred for 1h at -78C, and then diluted with petrol (100 ml) and filtered through a plastic cloumn containg neutral alumina (100 g). The resulting solution was stirred over anhydrous KF to remove HF residues and concetrated under reduced pressure to afford a crude oil (15.02g). The was dissolved in DMF (200 ml) and NBS (45g, 255 mmol) was added portionwise. The resulting mixture was stirred for 16h in the the absence of light. The DMF was distilled under reduced pressure and the crude residue filtered thorugh silica (eluent: petrol) to afford a crude brown oil (10.5g). This was further purified by bulb-to-bulb distillation (100C, 0.4 mbar) to afford **8** (5.71g, 27%). Purity HPLC + .MS (El) M = 362 (t, M⁺). The ¹H, ¹³C and ¹⁹F NMR were consistent with the proposed structure.

### Reference Example 4

### Poly[3-(1,1-difluoroalkyl)thiophene (4)

In a dry weighed schlenk tube was added a solution of Rieke zinc in THF (aldrich) and the THF removed under reduced pressurd to afford 0.21g (3.2 mmol) of active zinc. This was redissolved in dry THF (15 ml) and cooled to -78C. 2,5-Dibromo-3-(1',1'-difluorohexyl)thiophene (0.88g, 2.43 mmol) was added via a syringe and the reaction allowed to warm to RT over 20 min. Stirred for 2 h at RT until complete mono zic insertion was observed by GC (quenched sample). Stirring was halted and the ecess zinc allowed to settle. In a separate schlenk flask Ni(dmpe)Cl₂ (9.7 mg, 1 mol%) was stirred in THF (5 ml). The organozinc reagent was transferred via cannula into the solution of catalyst at 0°C. The solution was warmed to 60°C for 96 h, cooled and poured into methanol (50 ml) containing 10% HCI (10 ml). The resulting suspension was filtered and washed with methanol (3 x 10 ml), petrol (3 x 10 ml), and acetone (3 x 10 ml) to remove inorganics and low MW material. The resulting solid was dissolved in THF and reprecipitated into methanol, filtered and dried to afford poly[3-(1,1-difluoroalkyl)-thiophene as a red solid (101 mg, 18%). Gel permeation chromatography shows Mw 7300 Daltons, Mn 5800 Daltons. λₘₐₓ (THF) 438 nm.

## Claims

1. Mono-, oligo- and polymers selected of formula 11
R⁵-[(Y)ₐ-(D)_{b}-(Z)_{c}]ₙ-R⁶ l1
wherein
D is a 3-(1,1-difluoroalkyl)thiophene group of formula II 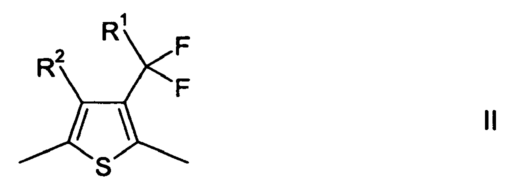
R¹ is H, straight chain, branched or cyclic alkyl with 1 to 20 C-atoms, which may be unsubstituted, mono- or partially-substituted by F, Cl, Br, I, -CN or -OH, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, or optionally substituted aryl or heteroaryl,
R² is H, F, straight chain, branched or cyclic alkyl with 1 to 20 C-atoms, which may be unsubstituted, mono- or poly-substituted by F, Cl, Br, I, -CN or -OH, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -CO-, -CO-S-, -CH=CH- or -C≡C- in such a manner that O and/or S atoms are not linked directly to one another, or optionally substituted aryl or heteroaryl,
R⁰ and R⁰⁰ are independently of each other H or alkyl with 1 to 12 C-atoms,
Y and Z are independently of each other -CX¹=CX²-, -C≡C-, or optionally substituted arylene or heteroarylene,
X¹ and X² are independently of each other H, F, Cl or CN, and
a, b and c are independently of each other 0 or 1, with a + b + c > 0, and wherein in at least one recurring unit b is 1,
n is an integer from 1 to 5000,
R⁵ and R⁶ are independently of each other H, halogen, Sn(R°)₃ or straight chain, branched or cyclic alkyl with 1 to 20 C-atoms, which may be unsubstituted, mono- or poly-substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, or optionally substituted aryl or heteroaryl, or denote P-Sp-X,
P is a polymerisable or reactive group,
Sp is a spacer group or a single bond, and
X is -O-, -S-, -OCH₂-, -CH₂O-, -CO-, -COO-, -OCO-, -OCO-O-, -CO-NR⁰-, -NR⁰-CO-, -OCH₂-, -CH₂O-, - SCH₂-, -CH₂S-, -CH=CH-COO-, -OOC-CH=CH- or a single bond,
wherein the recurring units [(Y)ₐ-(D)_{b}-(Z)_{c}] can be identical or different,
**characterized in that** one or both of R⁵ and R⁶ denote P-Sp-X,
wherein P is selected from CH₂=CW¹-COO-, 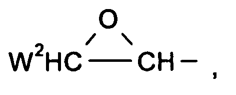 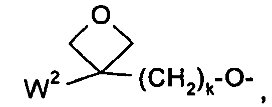 CH₂=CW²-(O)ₖ₁-, CH₃-CH=CH-O-, HO-CW²W³-, HS-CW²W³-, HW²N-, HO-CW²W³-NH-, CH₂=CW¹-CO-NH-, CH2=CH-(COO)ₖ₁-Phe-(O)ₖ₂-, Phe-CH=CH-, HOOC-, OCN- and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

2. Mono-, oligo- and polymers according to claim 1, wherein Sp is a linear or branched alkylene group having 1 to 20 C atoms, in particular 1 to 12 C atoms, in which, in addition, one or more non-adjacent CH₂ groups may be replaced by -O-, -S-, -NH-, - N(CH₃)-, -CO-, -O-CO-, -S-CO-, -O-COO-, -CO-S-, -CO-O-, - CH(halogen)-, -C(halogen)₂, -CH(CN)-, -CH=CH- or -C≡C-, or a siloxane group.

3. Mono-, oligo- and polymers according to claim 1 or 2, wherein R¹ is an alkyl group with 2 to 15 C-atoms, which may contain one CF₂- or CF₃-group, without being perfluorinated.

4. Mono-, oligo- and polymers according to at least one of claims 1 to 3, wherein R² is H, F or an optionally substituted alkyl group with 1 to 20 C atoms which is not perfluorinated

5. Mono-, oligo- and polymers according to at least one of claims 1 to 4, selected from the following formulae 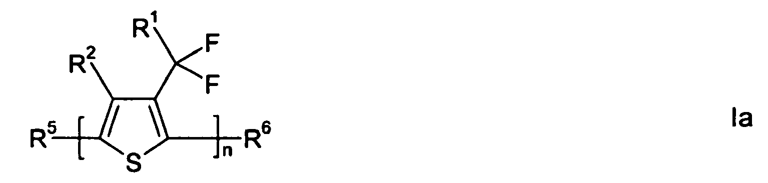 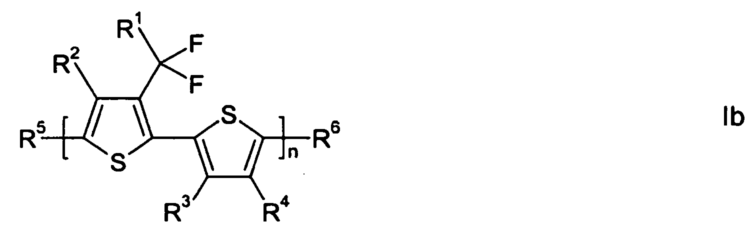 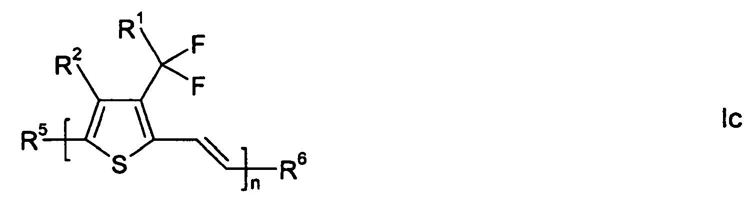 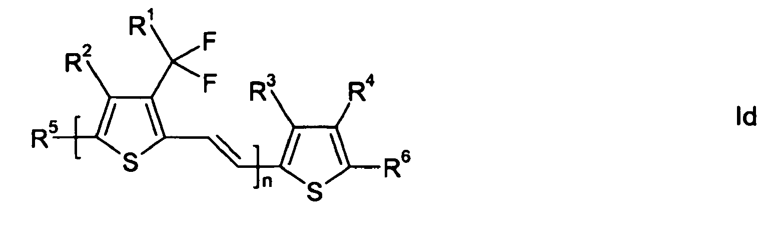 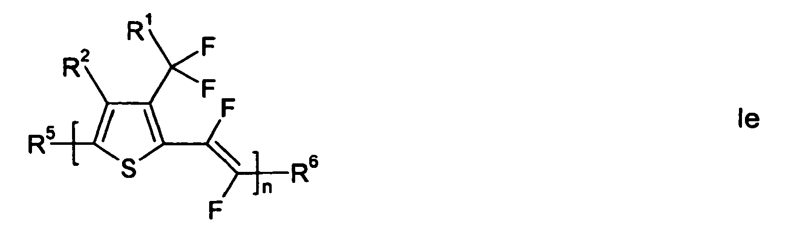 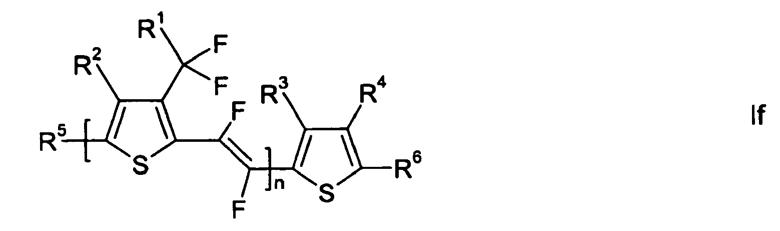 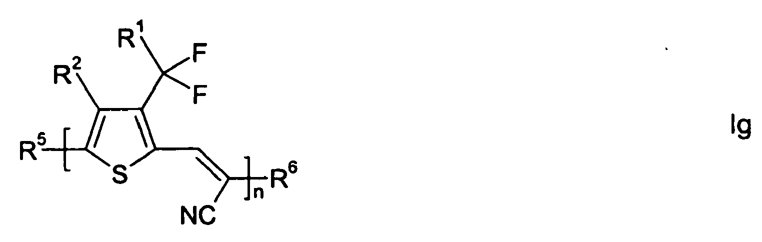 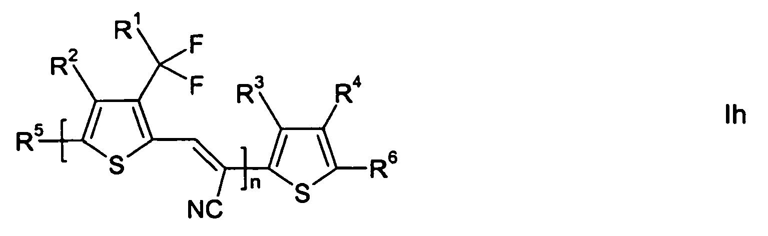 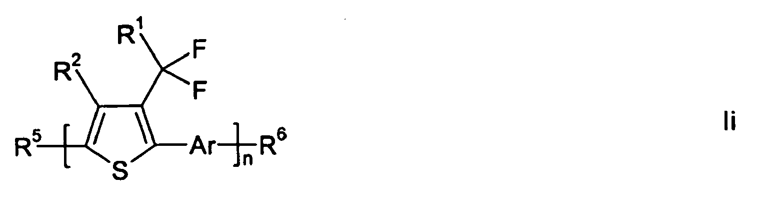 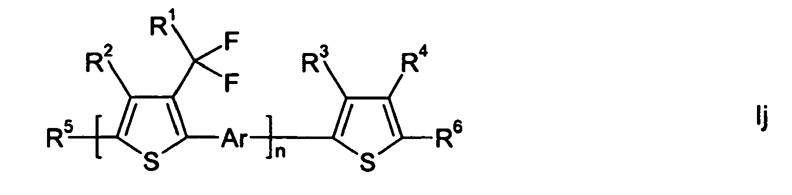 wherein
R¹ and R² have the meanings given in claim 1,
R⁵, R⁶ and n have the meanings given in claim 1,
R³, R⁴ independently of each other have one of the meanings of R² given in claim 1,
Ar is a bivalent mono-, bi- or tricyclic aromatic or heteroaromatic group with up to 25 C atoms that may also comprise condensed rings and is optionally mono- or poly-substituted with F, Cl and/ or groups R² as defined in claim 1.

6. Mono-, oligo- and polymers according to at least one of claims 1 to 5, wherein n is an integer from 1 to 15.

7. Polymerisable liquid crystal material comprising one or more mono-, oligo- or polymers according to at least one of claims 1 to 6 comprising at least one polymerisable group, and optionally one or more further polymerisable compounds, wherein at least one of said mono-, oligo- and polymers and/ or said further polymerisable compounds is mesogenic or liquid crystalline.

8. Anisotropic polymer film with charge transport properties obtainable from a polymerisable liquid crystal material according to claim 7 that is aligned in its liquid crystal phase into macroscopically uniform orientation and polymerised or crosslinked to fix the oriented state.

9. Side chain liquid crystal polymer obtained by polymerisation of one or more mono- or oligomers or a polymerisable material according to at least one of claims 1 to 7 or by grafting one or more mono- or oligomers or a polymerisable material according to at least one of claims 1 to 7 to a polymer backbone in a polymeranaloguous reaction, optionally with one or more additional mesogenic or non-mesogenic comonomers.

10. Use of mono-, oligo- and polymers, a polymerisable material or a polymer according to at least one of claims 1 to 9 as semiconductors or charge transport materials in optical, electrooptical or electronic devices, like for example components of integrated circuitry, field effect transistors (FET) for example as thin film transistors in flat panel display applications or for Radio Frequency Identification (RFID) tags, and in semiconducting components for organic light emitting diode (OLED) applications, electroluminescent display devices, backlights, photovoltaic, sensor or electrophotographic devices.

11. Field effect transistor (FET), OLED, electroluminescent device, RFID tag, backlight, photovoltaic or sensor device, or electrophotographic recording device comprising one or more mono-, oligo- or polymers, a polymerisable material or a polymer according to at least one of claims 1 to 9.

12. Security marking or device comprising one or more mono-, oligo- or polymers, a polymerisable material or a polymer according to at least one of claims 1 to 9 or a FET or RFID tag according to claim 11.

13. Mono-, oligo- and polymers, polymerisable material or polymer according to at least one of claims 1 to 9, which are oxidatively or reductively doped to form conducting ionic species.

14. Charge injection layer, planarising layer, antistatic film or conducting substrate or pattern for electronic applications or flat panel displays, comprising mono-, oligo- or polymers, a polymerisable material or polymer according to claim 13.

## Patentansprüche

1. Mono-, Oligo- und Polymere ausgewählt aus der Formel I1
R⁵-[(Y)ₐ-(D)_{b}-(Z)_{c}]ₙ-R⁶ 1 I1
worin
D eine 3-(1,1-Difluoralkyl)thiophengruppe der Formel II 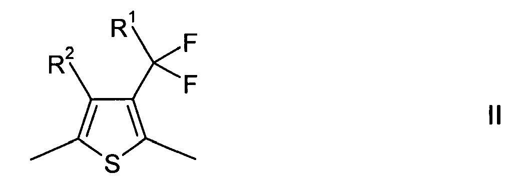 bedeutet,
R¹ H, geradkettiges, verzweigtes oder cyclisches Alkyl mit 1 bis 20 C-Atomen, welches unsubstituiert oder einfach oder teilweise durch F, CI, Br, I, -CN oder -OH substituiert ist, wobei auch eine oder mehrere nicht benachbarte CH₂-Gruppen jeweils unabhängig voneinander so durch -O-, -S-, -NH-, -NR°-, -SiR⁰R⁰⁰-, -CO-, -COO-, -OCO-, -OCO-O-, -S-CO-, -CO-S-, -CH=CH- oder -C≡C- ersetzt sein können, dass O- und/oder S-Atome nicht direkt miteinander verknüpft sind, oder gegebenenfalls substituiertes Aryl oder Heteroaryl bedeutet,
R² H, F, geradkettiges, verzweigtes oder cyclisches Alkyl mit 1 bis 20 C-Atomen, welches unsubstituiert oder ein- oder mehrfach durch F, Cl, Br, I, -CN oder -OH substituiert ist, wobei auch eine oder mehrere nicht benachbarte CH₂-Gruppen jeweils unabhängig voneinander so durch -O-, -S-, -NH-, -NR°-, -SiR⁰R⁰⁰-, -CO-, -COO-, -OCO-, -OCO-O-, -CO-, -CO-S-, -CH=CH- oder -C≡C- ersetzt sein können, dass O- und/oder S-Atome nicht direkt miteinander verknüpft sind, oder gegebenenfalls substituiertes Aryl oder Heteroaryl bedeutet,
R⁰ und R⁰⁰ unabhängig voneinander H oder Alkyl mit 1 bis 12 C-Atomen bedeuten,
Y und Z unabhängig voneinander -CX¹=CX²-, -C≡C- oder gegebenenfalls substituiertes Arylen oder Hetero-arylen bedeuten,
X¹ und X² unabhängig voneinander H, F, Cl oder CN bedeuten und
a, b und c unabhängig voneinander 0 oder 1 bedeuten, wobei a + b + c > 0 und wobei in mindestens einer wiederkehrenden Einheit b 1 bedeutet,
n eine ganze Zahl von 1 bis 5000 bedeutet,
R⁵ und R⁶ unabhängig voneinander H, Halogen, Sn(R°)₃ oder geradkettiges, verzweigtes oder cyclisches Alkyl mit 1 bis 20 C-Atomen, welches unsubstituiert oder ein- oder mehrfach durch F, Cl, Br, I oder CN substituiert ist, wobei auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -CH=CH- oder -C≡C- ersetzt sein können, dass O- und/oder S-Atome nicht direkt miteinander verknüpft sind, oder gegebenenfalls substituiertes Aryl oder Heteroaryl bedeuten, oder P-Sp-X darstellen,
P eine polymerisierbare oder reaktive Gruppe bedeutet,
Sp eine Spacergruppe oder eine Einfachbindung bedeutet und
X -O-, -S-, -OCH₂-, -CH₂O- -CO-, -COO-, -OCO-, -OCO-O-, -CO-NR⁰-, -NR⁰-CO-, -OCH₂-, -CH₂O-, -SCH₂-, -CH₂S-, -CH=CH-COO-, -OOC-CH=CH- oder eine Einfachbindung bedeutet,
worin die wiederkehrenden Einheiten [(Y)ₐ-(D)_{b}-(Z)_{c]} gleich oder verschieden sein können,
**dadurch gekennzeichnet, dass** eines oder beide von R⁵ und R⁶ P-Sp-X darstellen,
worin P ausgewählt ist aus CH₂=CW¹-COO-, 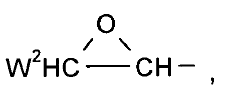 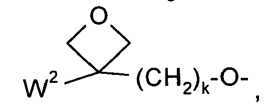 CH₂=CW²-(O)ₖ₁-, CH₃-CH=CH-O-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN- und W⁴W⁵W⁶Si-, wobei W¹ H, Cl, CN, Phenyl oder Alkyl mit 1 bis 5 C-Atomen, insbesondere H, Cl oder CH₃ bedeutet, W² und W³ unabhängig voneinander H oder Alkyl mit 1 bis 5 C-Atomen, insbesondere Methyl, Ethyl oder n-Propyl bedeuten, W⁴, W⁵ und W⁶ unabhängig voneinander Cl, Oxaalkyl oder Oxacarbonylalkyl mit 1 bis 5 C-Atomen bedeuten, Phe 1,4-Phenylen bedeutet und k₁ und k₂ unabhängig voneinander 0 oder 1 bedeuten.

2. Mono-, Oligo- und Polymere nach Anspruch 1, worin Sp eine lineare oder verzweigte Alkylengruppe mit 1 bis 20 C-Atomen, insbesondere 1 bis 12 C-Atomen bedeutet, in der zusätzlich eine oder mehrere nicht benachbarte CH₂-Gruppen durch -O-, -S-, -NH-, -N(CH₃)-, -CO-, -O-CO-, -S-CO-, -O-COO-, -CO-S-, -CO-O-, -CH(Halogen)-, -C(Halogen)₂, -CH(CN)-, -CH=CH- oder -C=C- ersetzt sein können, oder eine Siloxangruppe bedeutet.

3. Mono-, Oligo- und Polymere nach Anspruch 1 oder 2, worin R¹ eine Alkylgruppe mit 2 bis 15 C-Atomen bedeutet, welche eine CF₂- oder CF₃-Gruppe enthalten kann, ohne perfluoriert zu sein.

4. Mono-, Oligo- und Polymere nach mindestens einem der Ansprüche 1 bis 3, worin R² H, F oder eine gegebenenfalls substituierte Alkylgruppe mit 1 bis 20 C-Atomen bedeutet, welche nicht perfluoriert ist.

5. Mono-, Oligo- und Polymere nach mindestens einem der Ansprüche 1 bis 4, ausgewählt aus den folgenden Formeln 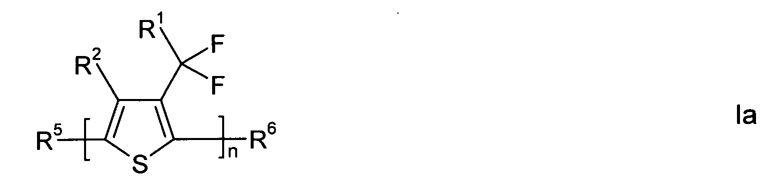 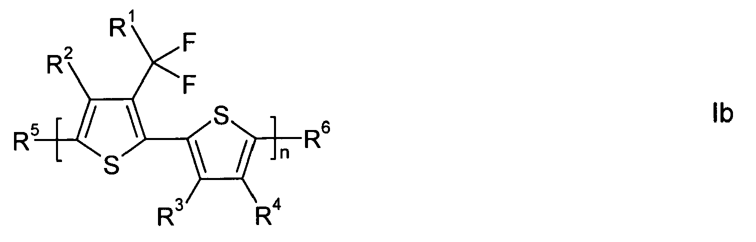 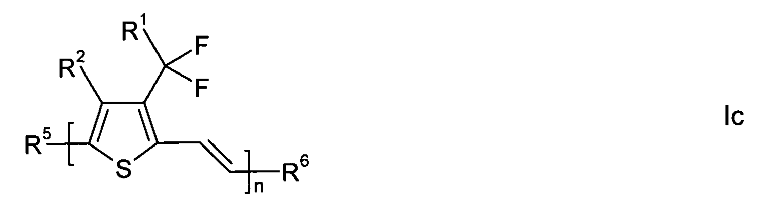 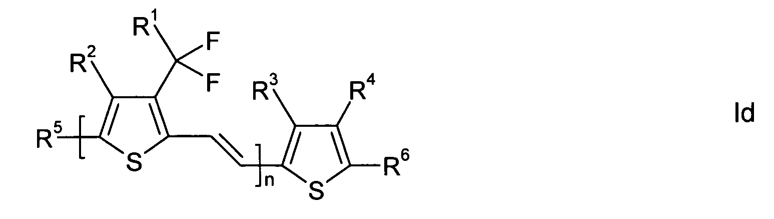 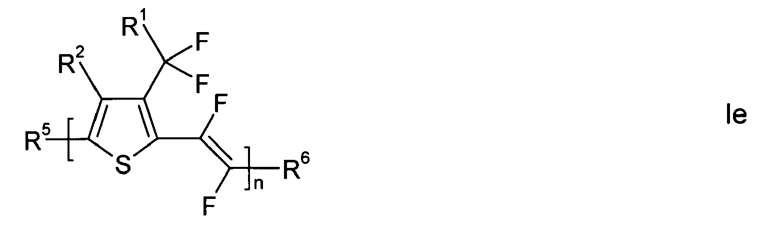 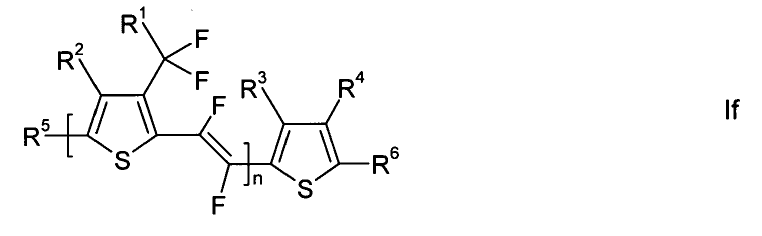 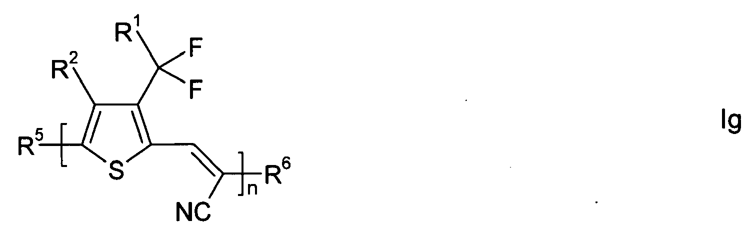 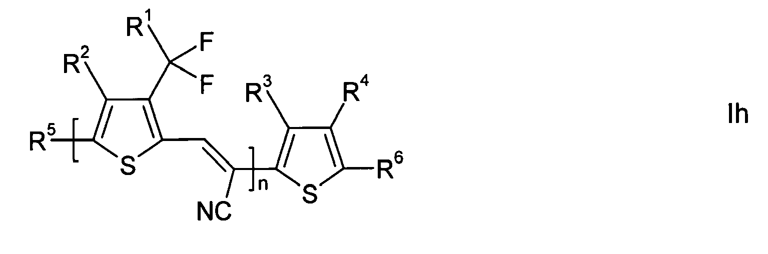 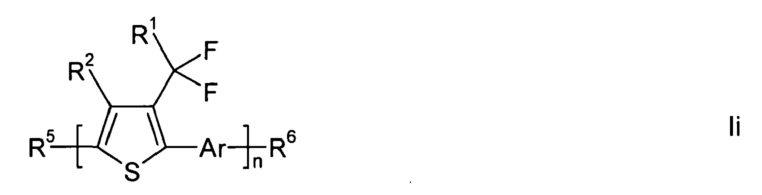 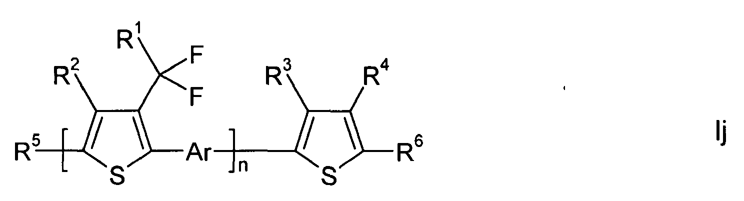 worin
R¹ und R² die in Anspruch 1 angegebenen Bedeutungen besitzen,
R⁵, R⁶ und n die in Anspruch 1 angegebenen Bedeutungen besitzen,
R³, R⁴ unabhängig voneinander eine der in Anspruch 1 angegebenen Bedeutungen von R² besitzen,
Ar eine zweiwertige mono-, bi- oder tricyclische aromatische oder heteroaromatische Gruppe mit bis zu 25 C-Atomen bedeutet, die auch kondensierte Ringe enthalten kann und gegebenenfalls ein- oder mehrfach mit F, Cl und/oder Gruppen R² wie in Anspruch 1 definiert substituiert ist.

6. Mono-, Oligo- und Polymere nach mindestens einem der Ansprüche 1 bis 5, worin n eine ganze Zahl von 1 bis 15 bedeutet.

7. Polymerisierbares Flüssigkristallmaterial enthaltend ein oder mehrere Mono-, Oligo- oder Polymere nach mindestens einem der Ansprüche 1 bis 6 enthaltend mindestens eine polymerisierbare Gruppe, und gegebenenfalls eine oder mehrere weitere polymerisierbare Verbindungen, worin mindestens eines der Mono-, Oligo- und Polymere und/oder der weiteren polymerisierbaren Verbindungen mesogen oder flüssigkristallin ist.

8. Anisotrope Polymerfolie mit Ladungstransporteigenschaften erhältlich aus einem polymerisierbaren Flüssigkristallmaterial nach Anspruch 7, welches in seiner Flüssigkristallphase in makroskopisch einheitliche Ausrichtung orientiert und polymerisiert oder vernetzt wird, um den ausgerichteten Zustand zu fixieren.

9. Seitenketten-Flüssigkristallpolymer erhalten durch Polymerisation eines oder mehrerer Mono- oder Oligomere oder eines polymerisierbaren Materials nach mindestens einem der Ansprüche 1 bis 7 oder durch Pfropfen eines oder mehrerer Mono- oder Oligomere oder eines polymerisierbaren Materials nach mindestens einem der Ansprüche 1 bis 7 auf ein Polymerrückgrat in einer polymeranalogen Reaktion, gegebenenfalls mit einem oder mehreren zusätzlichen mesogenen oder nicht mesogenen Comonomeren.

10. Verwendung von Mono-, Oligo- und Polymeren, einem polymerisierbaren Material oder einem Polymer nach mindestens einem der Ansprüche 1 bis 9 als Halbleiter oder Ladungstransportmaterialien in optischen, elektrooptischen oder elektronischen Vorrichtungen, wie zum Beispiel Bauteilen von integrierten Schaltungen, Feldeffekttransistoren (FET) z.B. als Dünnschichttransistoren in Flachbildschirm-Anwendungen oder für RFID-Tags (Radio Frequency Identification), und in Halbleiterbauteilen für Anwendungen mit organischen Leuchtdioden (organic light emitting diode - OLED), Elektrolumineszenzanzeigevorrichtungen, Hintergrundbeleuchtungen, Photovoltaik-, Sensor- oder elektrophotographischen Vorrichtungen.

11. Feldeffekttransistor (FET), OLED, Elektrolumineszenzvorrichtung, RFID-Tag, Hintergrundbeleuchtung, Photovoltaik- oder Sensorvorrichtung oder elektrophotographische Aufzeichnungsvorrichtung enthaltend ein oder mehrere Mono-, Oligo- oder Polymere, ein polymerisierbares Material oder ein Polymer nach mindestens einem der Ansprüche 1 bis 9.

12. Sicherheitsmarkierung oder -vorrichtung enthaltend ein oder mehrere Mono-, Oligo- oder Polymere, ein polymerisierbares Material oder ein Polymer nach mindestens einem der Ansprüche 1 bis 9 oder einen FET oder ein RFID-Tag nach Anspruch 11.

13. Mono-, Oligo- und Polymere, ein polymerisierbares Material oder Polymer nach mindestens einem der Ansprüche 1 bis 9, die oxidativ oder reduktiv dotiert sind, um leitende Ionenspezies zu bilden.

14. Ladungsinjektionsschicht, Planarisierungsschicht, antistatische Folie oder leitendes Substrat oder leitende Struktur für Elektronikanwendungen oder Flachbildschirme, enthaltend Mono-, Oligo- oder Polymere, ein polymerisierbares Material oder Polymer nach Anspruch 13.

## Revendications

1. Mono-, oligo- et polymères sélectionnés de formule I1
R⁵-[(Y)ₐ-(D)_{b}-(Z)_{c}]ₙ-R⁶ I1
dans laquelle
D est un groupe 3-(1,1-difluoroalkyl)thiophène de formule II 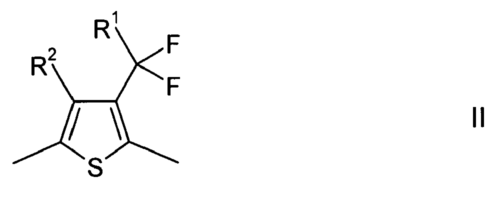
R¹ est H, alkyle en chaîne droite, ramifié ou cyclique comportant de 1 à 20 atomes de C, lequel peut être non substitué, monosubstitué ou partiellement substitué par F, CI, Br, I, -CN ou -OH, étant entendu qu'il est également possible qu'un ou plusieurs groupes CH₂ non adjacents soient remplacés, dans chaque cas indépendamment les uns des autres, par -O-, -S-, -NH-, -NR⁰**-,** -SiR⁰R⁰⁰-, -CO-, -COO-, -OCO-, -OCO-O-, -S-CO-, -CO-S-, -CH=CH- ou -C≡C- de telle sorte que des atomes de O et/ou de S ne soient pas liés directement les uns aux autres, ou aryle ou hétéroaryle en option substitué,
R² est H, F, alkyle en chaîne droite, ramifié ou cyclique comportant de 1 à 20 atomes de C, lequel peut être non substitué, monosubstitué ou polysubstitué par F, CI, Br, I, -CN ou -OH, étant entendu qu'il est également possible qu'un ou plusieurs groupes CH₂ non adjacents soient remplacés, dans chaque cas indépendamment les uns des autres, par -O-, -S-, -NH-, -NR⁰-, -SiR⁰R⁰⁰-, -CO-, -COO-, -OCO-, -OCO-O-, -CO-, -CO-S-, -CH=CH- ou -C=C- de telle sorte que des atomes de O et/ou de S ne soient pas liés directement les uns aux autres, ou aryle ou hétéroaryle en option substitué,
R⁰ et R⁰⁰ sont, indépendamment l'un de l'autre, H ou alkyle comportant de 1 à 12 atomes de C,
Y et Z sont, indépendamment l'un de l'autre, -CX¹=CX²-, -C≡C-, ou arylène ou hétéroarylène en option substitué,
X¹ et X² sont, indépendamment l'un de l'autre, H, F, Cl ou CN, et
a, b et c sont, indépendamment les uns des autres, 0 ou 1, avec a + b + c > 0, et où, dans au moins une unité récurrente, b est 1,
n est un entier de 1 à 5000,
R⁵ et R⁶ sont, indépendamment l'un de l'autre, H, halogène, Sn(R⁰)₃ ou alkyle en chaîne droite, ramifié ou cyclique comportant de 1 à 20 atomes de C, lequel peut être non substitué, monosubstitué ou polysubstitué par F, CI, Br, I ou CN, étant entendu qu'il est également possible qu'un ou plusieurs groupes CH₂ non adjacents soient remplacés, dans chaque cas indépendamment les uns des autres, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou de S ne soient pas liés directement les uns aux autres, ou aryle ou hétéroaryle en option substitué, ou représentent P-Sp-X,
P est un groupe polymérisable ou réactif,
Sp est un groupe d'espaceur ou une liaison simple, et
X est -O-, -S-, -OCH₂-, -CH₂O-, -CO-, -COO-, -OCO-, -OCO-O-, -CO-NR⁰-, -NR⁰-CO-, -OCH₂-, -CH₂O-, -SCH₂-, -CH₂S-, -CH=CH-COO-, -OOC-CH=CH- ou une liaison simple,
où les unités récurrentes [(Y)ₐ-(D)_{b}-(Z)_{c}] peuvent être identiques ou différentes,
**caractérisés en ce que** l'un de R⁵ et R⁶ ou les deux représentent P-Sp-X,
où P est sélectionné parmi CH₂=CW¹-COO-, 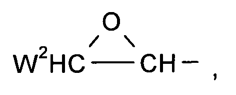 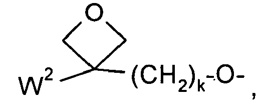 CH₂=CW²-(O)ₖ₁-, CH₃-CH=CH-O-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN- et W⁴W⁵W⁶Si, W¹ étant H, CI, CN, phényle ou alkyle comportant de 1 à 5 atomes de C, en particulier H, Cl ou CH₃, W² et W³ étant, indépendamment l'un de l'autre, H ou alkyle comportant de 1 à 5 atomes de C, en particulier méthyle, éthyle ou n-propyle, W⁴, W⁵ et W⁶ étant, indépendamment les uns des autres, CI, oxaalkyle ou oxacarbonylalkyle comportant de 1 à 5 atomes de C, Phe étant 1,4-phénylène et k₁ et k₂ étant, indépendamment l'un de l'autre, 0 ou 1.

2. Mono-, oligo- et polymères selon la revendication 1, où Sp est un groupe alkylène linéaire ou ramifié comportant de 1 à 20 atomes de C, en particulier de 1 à 12 atomes de C, où, en plus, un ou plusieurs groupes CH₂ non adjacents peut/peuvent être remplacé(s) par -O-, -S-, -NH-, -N(CH₃)-, -CO-, -O-CO-, -S-CO-, -O-COO-, -CO-S-, -CO-O-, -CH(halogène)-, -C(halogène)₂, -CH(CN)-, -CH=CH- ou -C≡C-, ou un groupe siloxane.

3. Mono-, oligo- et polymères selon la revendication 1 ou 2, où R¹ est un groupe alkyle comportant de 2 à 15 atomes de C, lequel peut contenir un groupe CF₂- ou CF₃-, sans qu'il soit perfluoré.

4. Mono-, oligo- et polymères selon au moins l'une des revendications 1 à 3, où R² est H, F ou un groupe alkyle en option substitué comportant de 1 à 20 atomes de C, lequel est non perfluoré.

5. Mono-, oligo- et polymères selon au moins l'une des revendications 1 à 4, sélectionnés parmi les formules qui suivent 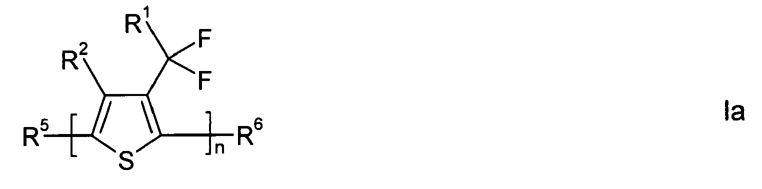 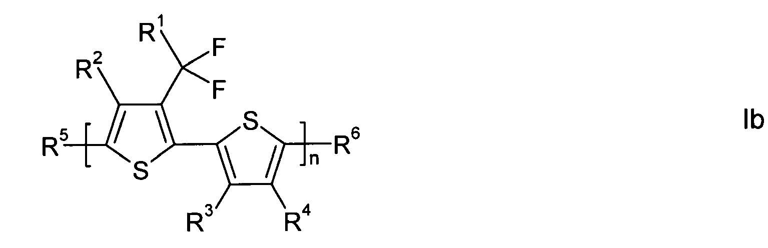 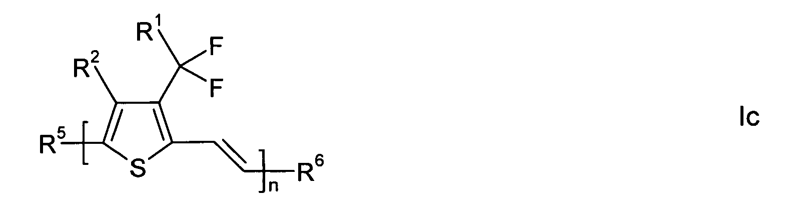 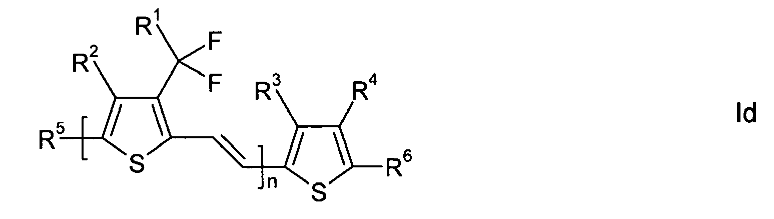 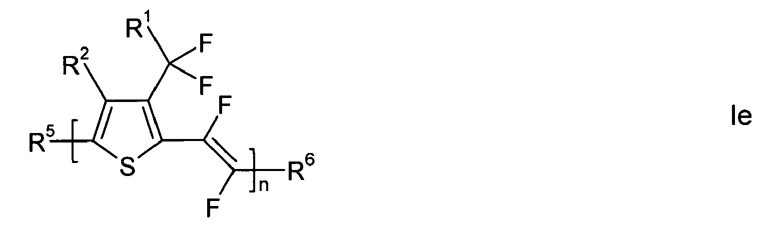 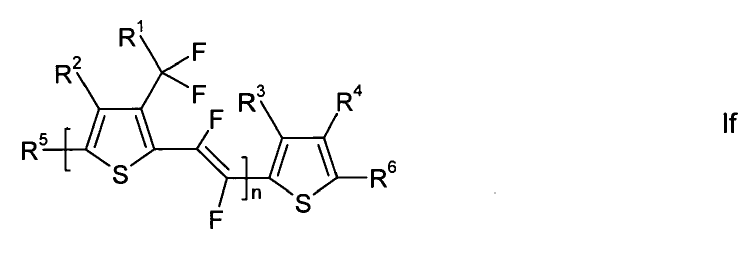 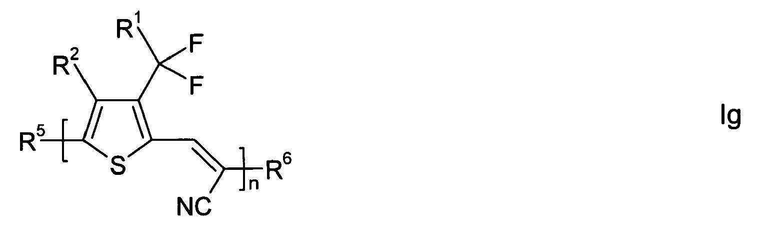 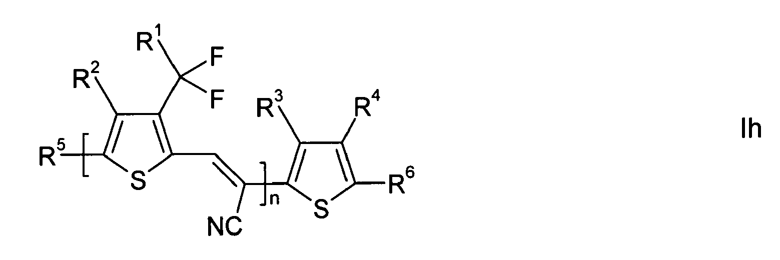 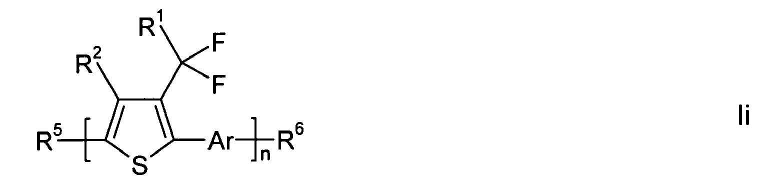 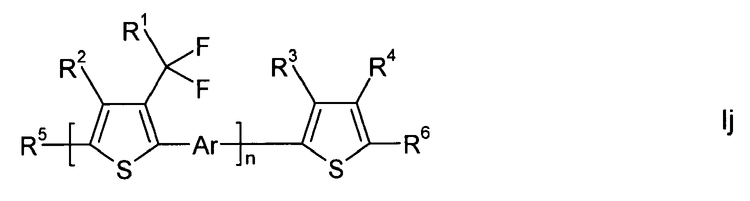 dans lesquelles
R¹ et R² présentent les significations données selon la revendication 1,
R⁵, R⁶ et n présentent les significations données selon la revendication 1,
R³, R⁴ indépendamment l'un de l'autre, présentent l'une des significations de R² données selon la revendication 1,
Ar est un groupe aromatique ou hétéroaromatique mono-, bi- ou tricyclique bivalent comportant jusqu'à 25 atomes de C, lequel peut également comprendre des cycles condensés et est en option mono- ou polysubstitué par F, Cl et/ou des groupes R² tels que définis selon la revendication 1.

6. Mono-, oligo- et polymères selon au moins l'une des revendications 1 à 5, où n est un entier de 1 à 15.

7. Matériau cristallin liquide polymérisable comprenant un ou plusieurs mono-, oligo- ou polymères selon au moins l'une des revendications 1 à 6 comprenant au moins un groupe polymérisable, et en option, un ou plusieurs autres composés polymérisables, où au moins l'un desdits mono-, oligo- et polymères et/ou desdits autres composés polymérisables est/sont cristallin(s) mésogène(s) ou liquide(s).

8. Film polymérique anisotrope présentant des propriétés de transport de charges pouvant être obtenu à partir d'un matériau cristallin liquide polymérisable selon la revendication 7 qui est aligné dans sa phase cristalline liquide selon une orientation macroscopiquement uniforme et qui est polymérisé ou réticulé pour fixer l'état orienté.

9. Polymère cristallin liquide à chaîne latérale obtenu par polymérisation d'un ou de plusieurs mono- ou oligomères ou d'un matériau polymérisable selon au moins l'une des revendications 1 à 7 ou par greffe d'un ou de plusieurs mono- ou oligomères ou d'un matériau polymérisable selon au moins l'une des revendications 1 à 7 sur une ossature polymérique selon une réaction polymèranalogue, en option avec un ou plusieurs comonomères mésogènes ou non mésogènes additionnels.

10. Utilisation de mono-, d'oligo- et de polymères, d'un matériau polymérisable ou d'un polymère selon au moins l'une des revendications 1 à 9 en tant que semiconducteurs ou matériaux de transport de charges dans des dispositifs optiques, électro-optiques ou électroniques, tels que par exemple des composants de circuit intégré, des transistors à effet de champ (FET) par exemple en tant que transistors à film mince dans des applications d'affichage à écran plat ou pour des étiquettes d'Identification Radio Fréquence (RFID), et dans des composants de semiconduction pour des applications de diodes émettrices de lumière organiques (OLED), des dispositifs d'affichage électroluminescents, des éclairages arrière, des dispositifs photovoltaïques, de capteur ou électrophotographiques.

11. Transistor à effet de champ (FET), OLED, dispositif électroluminescent, étiquette RFID, éclairage arrière, dispositif photovoltaïque ou de capteur, ou dispositif d'enregistrement électrophoto-graphique comprenant un ou plusieurs mono-, oligo- ou polymères, un matériau polymérisable ou un polymère selon au moins l'une des revendications 1 à 9.

12. Marquage de sécurité ou dispositif comprenant un ou plusieurs mono-, oligo- ou polymères, un matériau polymérisable ou un polymère selon au moins l'une des revendications 1 à 9 ou un FET ou une étiquette RFID selon la revendication 11.

13. Mono-, oligo- et polymères, matériau polymérisable ou polymère selon au moins l'une des revendications 1 à 9, lesquels sont dopés par oxydation ou par réduction afin de former des espèces ioniques conductrices.

14. Couche d'injection de charges, couche de planarisation, film antistatique ou substrat ou motif de conduction pour des applications électroniques ou des affichages à écran plat, comprenant des mono-, des oligo- ou des polymères, un matériau polymérisale ou un polymère selon la revendication 13.
